# EUROPEAN PATENT APPLICATION

(11) **EP 2 508 522 A1**
(43) Date of publication of application: **10.10.2012**
(21) Application number: 10832671.1
(22) Date of filing: 29.11.2010
(51) Int. Cl.: C07D 413/10, C07C 215/08, C07C 215/40, A61K 31/4245, A61P 9/12

(54) **AZILSARTAN ORGANIC AMINE SALTS, PREPARATION METHOD AND USE THEREOF**

(30) Priority: 30.11.2009 CN 200910246554
(71) Applicant: Jiangsu Hansoh Pharmaceutical Group Co., Ltd., Jiangsu 222047 (CN)
(72) Inventor: LU, Aifeng, Lianyungang Jiangsu 222047 (CN); YANG, Baohai, Lianyungang Jiangsu 222047 (CN)
(74) Representative: Bond, Christopher William
(86) International application number: PCT/CN2010/079222
(87) International publication number: WO 2011/063764

(57) **Abstract**

Azilsartan organic amine salts, the preparation method and the use thereof are disclosed. Specially, azilsartan organic amine salts represented by formula (I), their preparation method, pharmaceutical compositions containing a therapeutically effective amount of said compounds and their use for preparing antihypertensive medicines are disclosed.

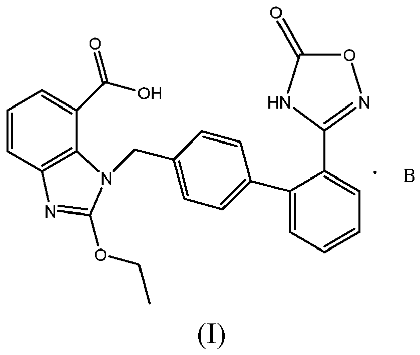

## Description

### FIELD OF THE INVENTION

The present invention relates to Azilsartan organic amine salts, preparation method thereof, and pharmaceutical compositions containing the therapeutically effective amount of said compounds and their use for preparing antihypertensives.

### BACKGROUND OF THE INVENTION

Azilsartan belongs to a selective antagonist of hypertension II1 receptor (AT1), it reduces blood pressure by selectively blocking the binding of hypertension II to the receptor of vascular smooth muscle AT1, which stop the vein shrinking induced by hypertension II.

There is carboxyl group in the molecular structure of Azilsartan, which leads to bad absorption of Azilsartan in vivo, so the Azilsartan is not easy to be prepared into pharmaceutical dosage forms. In order to improve the bioavailability, Azilsartan has be prepared to an ester by chemical modification, but its bioavailability is still not satisfied and its molecular structure becomes complex by such modification, which increases difficulty in syntheses.

### DETAILED DESCRIPTION OF THE INVENTION

This invention proves that the salts formed by Azilsartan and organic amines have better pharmacokinetics characters, higher bioavailability, and the salts are more suitable for normal preparation process.

The present invention provides Azilsartan organic amine salts represented by formula (I): 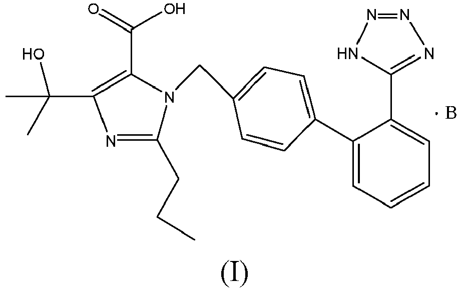 wherein, B is an organic amine selected from methylamine, dimethylamine, trimethylamine, ethylamine, diethylamine, triethylamine, ethanolamine, piperazidine, dibenzylethylenediamine, meglumine, tromethamine, tetramethyl quaternary ammonium, tetraethyl quaternary ammonium or choline; preferably ethanolamine 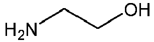 or choline

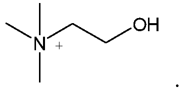

The present invention also provides a process for preparing the compounds mentioned above, which comprises adding acid form of Azilsartan and organic amine B separately into organic solvents, such as alcohol, to obtain corresponding salts at room temperature or under heating, wherein the solvents are selected from the group consisting of methanol, ethanol, propanol or isopropanol.

The present invention also provides a pharmaceutical composition for use in the treatment of hypertension, comprising a therapeutically effective amount of Azilsartan organic amine salts as an active ingredient and pharmaceutically acceptable carriers.

Furthermore, the invention also provides the use of Azilsartan organic amine salts and their pharmaceutical compositions in the preparation of an antihypertensive medicament.

In the preparation process of pharmaceutical compositions, it is important to prepare the drug into an appropriate dosage form conveniently, which is not only in the view of commercial available preparation but also in the view of preparing the pharmaceutical dosage forms containing the active compounds.

In another aspect, it is important to offer a reliable, reproducible and constant drug plasma concentration curve after administrating to a subject.

Other important factors include chemical durability, solid-state stability and storage life of the active ingredient. The drugs and the compositions containing the same can be preferably stored relatively for a long time with no obvious change in physical and chemical properties of their active components such as chemical composition, density, hygroscopicity and solubility.

It is also important to provide the drug as pure as possible.

Typically, a drug can offer following advantages: convenient handling, easy to prepare appropriate drug dosage forms and reliable solubility if the drug can be obtained in a stable form such as stable crystal form, which is well known by the person of skilled in the art.

Effective amount of the active ingredient in pharmaceutical dosage unit as described above will be nontoxic, preferably selected from the range 0.001∼100 mg/kg of total weight, more preferably 0.001∼50 mg/kg. When treating a subject with an Azilsartan organic amine salt, the selected dose is administrated preferably orally or parenterally. Preferred parenteral forms include topical, rectal, transdermal administration forms, injection and continuous infusion. Oral dosage units for human administration preferably contain from 0.05 to 3500 mg of active ingredient, most preferably from 0.5 to 1000 mg of active ingredient. Oral administration, which uses lower dosage is more preferred. Parenteral administration, at high dosages, however, also can be used when safe and convenient for the patient. The above dosages relates to the preferred amount of the active ingredient counted as the free acid.

It will be understood by one skilled in the art that the optimal quantity and period of dosages of the active ingredient for each individuals will depend on the nature and extent of the condition of the particular patient to be treated, the form, route and site of administration, , and such optimums can be determined by conventional techniques. It will also be appreciated by one skilled in the art that the optimal course of treatment, i.e., the number of doses of the active ingredient given per day for a defined number of days, can be ascertained by those skilled in the art using conventional testing methods.

The compounds claimed in the present invention can be administrated orally or parenterally, wherein the compounds can be prepared into tablets, pills, powder and granules used in different routes of administration. In above solid dosage forms, the active components mixed with at least one kind of inert diluent. According to conventional operation, oral dosage forms also include other substance such as lubricants, glidants and antioxidants besides inert diluent. If made into capsules, tablets and pills, dosage forms contain buffering agents. Tablets and pills can be made into sustained-release dosage forms too.

Although non-aqueous emulsions can be used, parenteral dosage forms of the present invention contain sterile aqueous solution and these dosage forms also contain adjuvants, for example antiseptics, wetting agents, penetrating agents, buffering agents, emulsifying agents and dispersants. The composition is sterilized by bacteria retaining filter, sterilizing agent, irradiation or heating.

Compared with Azilsartan and esters of Azilsartan, the salts of the present invention mainly have following advantages:
(1) The salts of the present invention are easily dissolved in the conventional solvents such as water, methanol, 0.1% hydrochloric acid and adapted to be prepared into conventional dosage forms.
(2) The salts of the present invention have improved stability.
(3) The salts of the present invention have better bioavailability.
(4) The preparation process of the salts of the present invention has the advantages of high yield, high purity, quick, convenience and low cost, wherein ethanolamine salt, choline salt are more advantageous in process routes.

### PREFERRED EMBODIMENTS

### Example 1

### Preparation of Azilsartan ethanolamine

Azilsartan (acid form) (1.37g) was added into methanol (30ml), then the mixture was added with ethanolamine (0.183g) at room temperature, heated to reflux. The insoluble substance was filtered out, and the filtrate was concentrated under reduced pressure to remove solvent. Acetone (20ml) was added into the residue, then the mixture was stirred for 2h, filtered, dried to obtain white solid 1.45g.
1H (DMSO-d6+D₂O) δ: 1.35 (t, 3H, CH₃), 2.83 (t, 2H, CH₂), 3.55 (t, 2H, CH₂), 4.52 (q, 2H, CH₂), 5.65 (s, 2H, CH₂), 7.03∼7.51 (m, 11H).

### Example 2

### Preparation of Azilsartan choline

Azilsartan (1.37g) and 46% aqueous solution of choline (0.79g) were added into ethanol (20ml) and the mixture was heated to reflux for 2h, stirred for one day at room temperature, dried under reduced pressure. Ethyl acetate (20ml) was added, then the mixture was stirred for 2h, filterated to obtain white solid.
1H NMR (DMSO-d6+D2O) δ: 1.35 (t, 3H, CH₃), 3.83 (t, 2H, CH₂), 3.55 (t, 2H, CH₂), 3.06 (s, 9H), 4.52 (q, 2H, CH₂), 5.65 (s, 2H, CH₂), 7.05∼7.50 (m, 11H).

### Experimental Example 1

### The effect of compounds of the present invention on blood pressure in angiotensin II-induced hypertensive rats

Male Sprague-Dawley rats (9-11 weeks old, CLEA Japan, Inc.) were anesthetized by pentobarbital solution (50 mg/kg, ip.) and prepared for surgery. The surgery procedure was as follows: made an incision through the skin on the abdomen and abdominal wall, separated the aorta and vein from the vena cava, occluded blood flow to allow introduction of a polyethylene (PE) tube filled with normal saline containing heparin (200 U/mL) into the vessel, passed the PE tube through the subcutaneous slits, and fixed the PE tube at the back of the neck. After recovery period, the rats were induced hypertension by intravenous administration of 100 ng/kg angiotensin II (AII). The PE tube was combined with a pressure transducer coupled to a blood pressure monitor amplifier (2238, NEC San-ei Instruments). The animals were enrolled based on average systolic blood pressure over 24 hr. All the animals with average systolic blood pressure less than 140 mmHg were excluded from this study, and the others were divided into 2 groups. The groups were single oral dosed equimolar amount of test articles. The rats were injected intravenously AII again after 24 hours, and their blood pressure were measured by the monitor. The rat blood pressure inhibition ratios after dosing were calculated. The test articles were formulated in 0.5% Methyl cellulose, and the dosing volume for all animals was 2 mL/kg. The results were expressed as Mean ± S.E. (Table 1).

**Table 1**

| Test compound | 24 hours after adminsitration |
|---|---|
| Product of example 1 (0.11mg/kg, p.o. n=4) | 57.4±6.7 |
| Product of example 2 (0.123mg/kg, p.o. n=6) | 54.3±5.1 |

Results of the experiment: The blood pressure significantly decreased following the treatments of present invention compounds, and the inhibition could persist a long time.

### Experimental Example 2

### The effect of compounds of the present invention on blood pressure in angiotensin II-induced hypertensive dogs

In this study, male Beagle dogs (weighting 12.0-14.7 kg, KITAYAMA LABES, CO., LTD.) were employed. The dogs were anesthetized by pentobarbital solution (50 mg/kg, ip) and prepared for surgery. The surgery procedure was as follows: made an endotracheal intubation for controlling the breathing, shaved the regions of femoral and back of the neck, fixed the dogs at the dorsal position, used isodine solution (MEIJI SEIKA KAISHA, LTD.) to sterilize the skins, made an incision through the skin on the right femoral region, separated the arteria femoralis, occluded blood flow to allow introduction of a mirror catheter (5 F, MILLER INDUSTRIES) and polyurethane tube into the aorta and vein, respectively, passed the tubes through the subcutaneous slits and fixed the tubes at the back, closed the wall and sutured the skin, and injected intramuscularly the penicillin G potassium (MEIJI SEIKA KAISHA, LTD., 40000 units) for sterilization. For post-operative care, animals were administered 40000 units penicillin G potassium intramuscularly once daily for 3 days. There was a recovery period before study ongoing.

The animals were singly housed in a small metabolic cage and fasted during the experiment. The mirror catheter was linked to a transducer unit (MILLER INDUSTRIES). Through a DC amplifier (N4777, NEC San-ei Instruments) and a blood pressure monitor amplifier (N4441, NEC San-ei Instruments), the blood pressure was recorded by a recorder (RECTI-HORIZ 8 K, NEC San-ei Instruments). In order to establish a hypertension model, the dogs were injected intravenously 100 ng/kg AII for 3 or 4 times before dosing test articles. The test articles were an equal molar dose suspended in 0.5% Methyl cellulose, and the dosing volume for all animals was 2 mL/kg. After administration, the blood pressure was measured by the monitor. The dog blood pressure inhibition ratios after dosing were calculated, and the results were expressed as Mean ± S.E. (Table 2).

**Table 2**

| Test compound | 10 hours after administration | 24 hours after administration |
|---|---|---|
| Product of example 1 (1.10mg/kg, p.o. n=6) | 82.7±7.3 | 61.6±4.8 |
| Product of example 2 (1.23 mg/kg, p.o. n=5) | 79.3±8.6 | 59.5±6.3 |

Results of the experiment: The blood pressure significantly decreased following the compounds of the present invention treatments. Also, the inhibitory effect sustained a long time.

Results of the experiment: the compounds of the present invention had a significantly long lasting and potent pharmacological action.

### Experimental Example 2

### Inhibitory effects of the compounds of the present invention on angiotensin II induced pressor response in dogs

For the experiment, male beagles (body weight 12.0-14.7 kg, KITAYAMA LABES, CO., LTD.) were used. They were anesthetized with sodium pentobarbital (50mg/kg, i.p.), and a tracheal tube was inserted for control of the airway. The femoral region and the back of the neck were shaved, and disinfected (Isodine solution, MEIJI SEIKA KAISHA, LTD.). The dog was fixed at the dorsal position and the right femoral region was incised. A mirror catheter (5 F, MILLER INDUSTRIES) was inserted and placed in the femoral artery and a polyurethane tube was placed in the femoral vein. The catheter and tube were passed through subcutaneously and fixed at the back region. The incised region was sutured thereafter and penicillin G potassium (MEIJI SEIKA KAISHA, LTD., 40000 units) was intramuscularly administered to prevent infection. Beginning from the next day, penicillin G potassium (40000 units) was administered once a day for 3 days. After 3 days for recovery, the dog was subjected to the experiment.

During the experiment, the dog was placed in a small metabolic cage.

For measurement, the mirror catheter inserted in the femoral artery was connected to a transducer unit (MILLER INDUSTRIES), and the systemic blood pressure (average blood pressure) was recorded on a recorder (RECTI-HORIZ 8 K, NEC San-ei Instruments) through a DC amplifier (N4777, NEC San-ei Instruments) and a blood pressure monitor amplifier (N4441, NEC San-ei Instruments). The polyurethane tube inserted in the femoral vein was fixed outside the cage and used for administration of AII (PEPTIDE INSTITUTE, INC.). The experiment was conducted under fasting and AII (100ng/kg, i.v.) was administered 3 or 4 times before administration of a test compound to confirm stabilization of the vasopressor response. A dose of the test compound corresponding to an equimolar amount of compound A was suspended in 0.5% methylcellulose and orally administered at a volume of 2 mL/kg. After drug administration, AII was administered at each time point of measurement and an increase in the blood pressure was measured, based on which the inhibition rate against the blood pressure value before administration was calculated. The results are shown as mean±SEM (Table 2).

Results of the experiment: the compounds of the present invention had a significantly long lasting and potent pharmacological action.

## Claims

1. Azilsartan organic amine salts represented by formula (I): 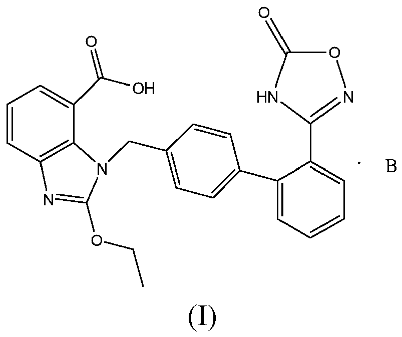 wherein, B is an organic amine.

2. The Azilsartan organic amine salts of claim 1, wherein B is selected from the group consisting of methylamine, dimethylamine, trimethylamine, ethylamine, diethylamine, triethylamine, ethanolamine, piperazidine, dibenzylethylenediamine, meglumine, tromethamine, tetramethyl quaternary ammonium, tetraethyl quaternary ammonium or choline.

3. The Azilsartan organic amine salts of claim 1, wherein B is ethanolamine represented by formula (II)

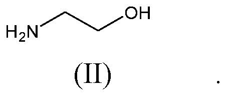

4. The Azilsartan organic amine salts of claim 1, wherein B is choline represented by formula (III)

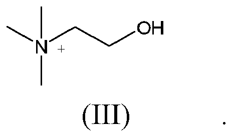

5. A process for preparing the Azilsartan organic amine salts of any one of claim 1 to 4, which comprises adding acid of Azilsartan and organic amine B separately into alcohol organic solvents to obtain corresponding salts at room temperature or under heating.

6. The process of claim 5, wherein said alcohol organic solvents are selected from the group consisting of methanol, ethanol, propanol or isopropanol.

7. A pharmaceutical composition for use in the treatment of hypertension, comprising a therapeutically effective amount of Azilsartan organic amine salts of any one of claim 1 to 4 as an active ingredient and pharmaceutically acceptable carriers.

8. Use of the Azilsartan organic amine salts of any one of claims 1 to 4, or the pharmaceutical composition of claim 7 in the preparation of an antihypertensive medicament.
